# EUROPEAN PATENT SPECIFICATION

(11) **EP 3 302 619 B1**
(45) Date of publication and mention of the grant of the patent: **04.11.2026**
(21) Application number: 16732062.1
(22) Date of filing: 03.06.2016
(51) Int. Cl.: A61M 1/36, A61F 2/06

(54) **A LOW BLEED IMPLANTABLE PROSTHESIS WITH A TAPER**
IMPLANTIERBARE PROTHESE MIT GERINGEM BLUTEN MIT VERJÜNGUNG
PROTHÈSE IMPLANTABLE À FAIBLE PERTE AVEC UNE CONICITÉ

(30) Priority: 05.06.2015 US 201562171694 P
(43) Date of publication of application: 11.04.2018
(73) Proprietor: W. L. Gore & Associates, Inc., Newark, DE 19711 (US)
(72) Inventor: SCHLAUD, Marc A., Newark, Delaware 19711 (US); STRINGER, Timothy T., Newark, Delaware 19711 (US)
(74) Representative: HGF
(86) International application number: PCT/US2016/035857
(87) International publication number: WO 2016/197017

(56) References cited:
- WO-A1-2006/085044
- WO-A1-96/07370
- US-A1- 2008 195 026

## Description

### FIELD

The present disclosure relates to implantable devices such as vascular grafts, and more particularly, relates to low bleed tubular vascular grafts with a tapered length portions such that opposing ends of the graft have different inside diameters.

### BACKGROUND

The use of implantable medical devices in the treatment of diseased vasculature and other body conduits has become commonplace in the medical field. These implantable devices can be used in applications where a low bleed implantable device is desired wherein the implantable device may be punctured (e.g., by suture needles and/or dialysis needles) and upon subsequent removal of the puncturing needle there is minimal fluid loss at the needle puncture site. Some of these implantable medical devices (e.g., a graft) may also be used in applications (e.g., dialysis) where a graft may benefit from a taper portion along its length, whereby one end of the graft has a larger inside diameter than an opposing end. A typical graft that has been designed for needle puncture may include an elastomeric material (e.g., silicone) that has a consistent wall thickness along the length of the graft. A typical graft, for example, a porous expanded polytetrafluoroethylene (ePTFE) graft that has been manufactured with a taper has a wall thickness at large end of the taper that is less than a wall thickness at a small end of the taper, therefore potentially limiting flexibility.

These tapered grafts and low bleed grafts have functioned well in many applications, particularly for dialysis applications. These previously known grafts still have limitations and leave room for improvements, especially in difficult applications such as where a low bleed graft and a taper is desired. Therefore, it remains desirable to provide a low bleed taper graft that has desirable handling attributes. There remains a need among vascular surgeons for such a graft.

WO 2006/085044 A1 relates to an artificial blood vessel for use in a region of a living body which needs to be repeatedly pierced with a needle. The artificial blood vessel includes a main section having a vessel wall which includes a laminated assembly comprising an inner layer, an outer layer , and an intermediate layer positioned therebetween, and a pair of anastomotic sections disposed on the respective opposite ends of the main section. Each of the anastomotic sections has an intermediate layer thinner than the intermediate layer of the main section or is free of an intermediate layer, so that the anastomotic section has a vessel wall thinner than the main section.

### SUMMARY

The present disclosure describes an implantable medical device as set forth in the appended claims, such as a vascular graft, incorporating a taper length portion and having low bleed characteristics following puncture by and removal of a needle such as a dialysis needle or a suture needle. A vascular graft per this disclosure may comprise a tubular structure having a first (Inner) layer, an intermediate layer, an optional second (outer) layer, and a length wherein said intermediate layer comprises an elastomer; wherein said tubular structure has a proximal end with proximal inside and outside diameters, and a distal end with a distal inside and outside diameters, wherein the distal inner and outer diameters are greater than the proximal inside and outside diameters respectively, and there is a transition length portion located between the proximal diameters and the distal diameters; and wherein the intermediate layer has a variable thickness along the length of the tubular structure and particularly along the transition length portion, wherein the thickness of the intermediate layer is greater at the distal end than it is at the proximal end. The first inner layer and the second outer layer may, in contrast to the intermediate layer (and counterintuitively), also be of variable thickness along the transition length portion but vary from being thicker at the proximal end to thinner at the distal end.

It is apparent that additional layers of materials as desired may be added to the three layer constructs described herein, resulting in constructs having four, five, six or more layers. In one embodiment, a first, inner layer may be a porous (e.g., ePTFE) material, an intermediate layer may be a non- porous (e.g., an elastomer) material, and an optional second, outer layer may be a porous (e.g., ePTFE) material. All materials should be suitable implantable materials. Suitable porous materials in addition to ePTFE may, for example, include implantable fabrics (e.g., polyethylene terephthalate) and porous polyurethanes and polyethylenes. Intermediate layer materials may include elastomers such as silicones and polyurethanes. These intermediate layer materials would most typically be nonporous materials, but porous versions of these same materials may also be used for this layer.

As layers extend along proximal, transition, and distal length portions; the materials making up the various layers may extend continuously and integrally between opposing ends of the graft. Alternatively, the various layers may be discreet, resulting in material boundaries between various length portions.

### BRIEF DESCRIPTION OF THE DRAWINGS

The accompanying drawings are included to provide a further understanding of the present disclosure and are incorporated in and constitute a part of this specification, illustrate embodiments of the present disclosure, and together with the description serve to explain the principles of the present disclosure.
FIG. 1 shows an isometric view of human anatomy with an implantable device.
FIGS. 2 shows a longitudinal cross section of a previously known implantable device with low bleed characteristics.
FIG. 3A shows a longitudinal cross section side view of an implantable device in accordance with the present disclosure.
FIG. 3B shows a longitudinal cross section side view of an implantable device in accordance with the present invention.
FIG. 3C shows a longitudinal cross section side view of an implantable device in accordance with the present invention.
FIG. 4 shows a transverse cross section taken at a designated location on the longitudinal cross section of Figure 3A.
FIG. 5 shows another transverse cross section taken at a designated location on the longitudinal cross section of Figure 3A.

### DETAILED DESCRIPTION

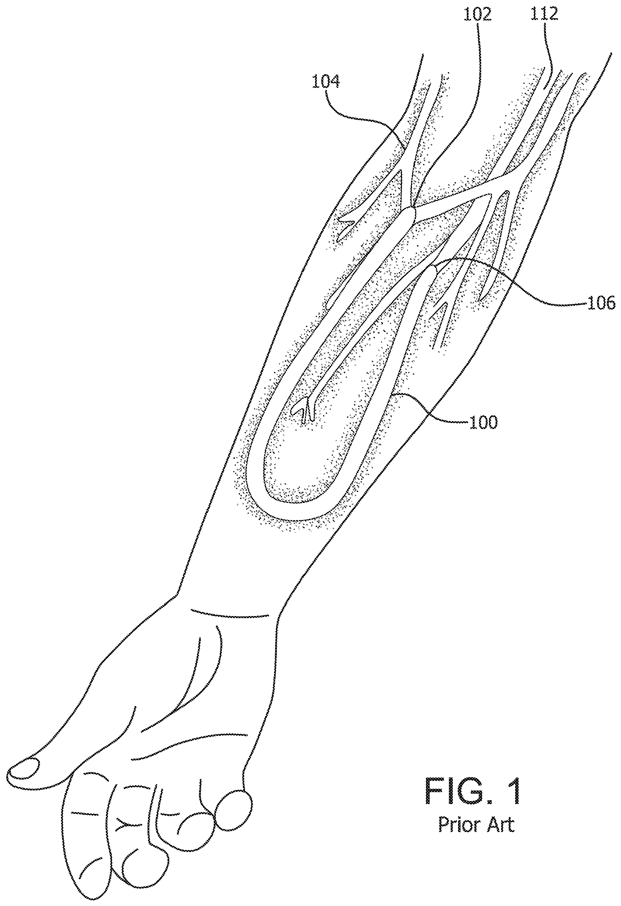

An implantable device (e.g., a vascular graft) in accordance with the present disclosure may be used in various applications. The implantable device 100 may be used in an arterial venous application (e.g., dialysis) as shown by example in Figure 1. The implantable device (e.g., graft) 100 may have a distal end 102 that may be in communication with venous vasculature 104 and a proximal end 106 that may be in communication with arterial vasculature 112 and a length between the proximal end and the distal end. The graft may have a tubular shape along the length of the graft. The implantable device 100 could also be a stent-graft or a hybrid of a stent-graft and a vascular graft.

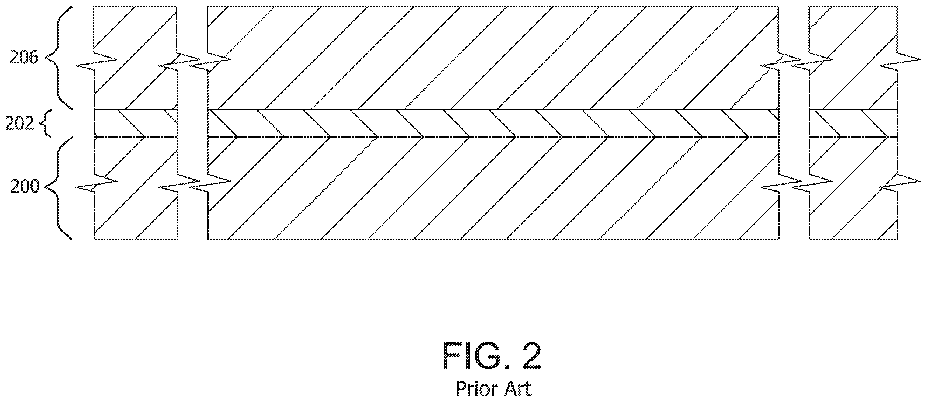

Figure 2 shows a longitudinal cross section of a portion of a length of previously known vascular graft with a first (inner) layer 200, an intermediate layer 202, and a second (outer) layer 206. The intermediate layer 202 (which may, for example, be an elastomer layer) is shown to have a constant wall thickness along the length of the vascular graft.

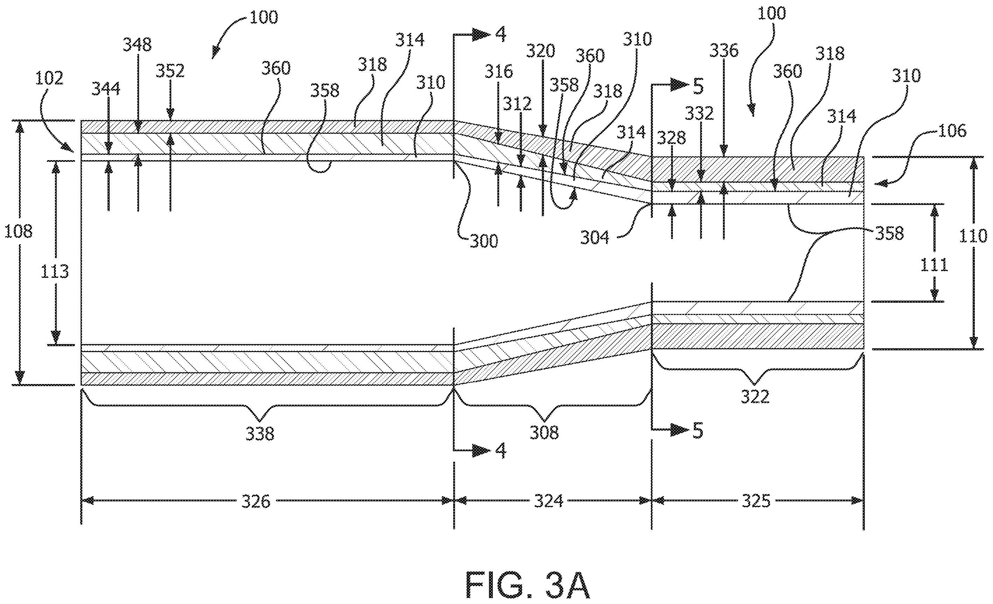

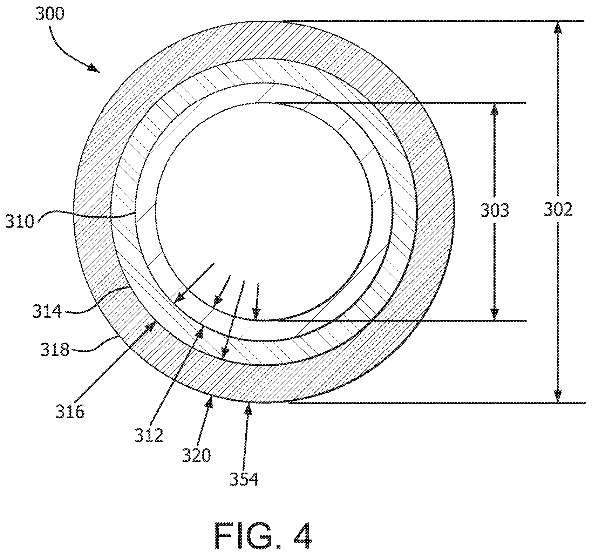

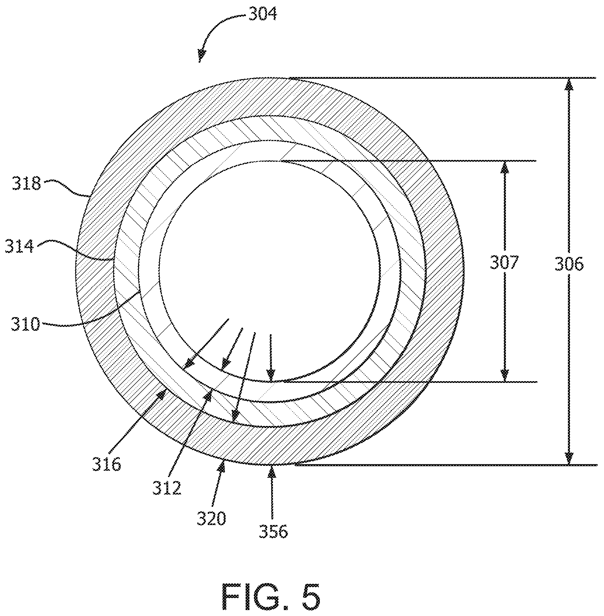

An implantable device 100 can have various configurations. As shown in the longitudinal cross section of Figures 3A-C and the transverse cross sections of Figure 4 and Figure 5, the implantable device 100 (e.g., a low bleed vascular graft) has a distal portion distal end 102 with a distal portion outer diameter 108 and a distal portion inside diameter 113. The implantable device 100, as shown in Figures 3A-C, has a proximal portion proximal end 106 with a proximal portion outer diameter 110 that is smaller than distal portion outer diameter 108 and a proximal portion inside diameter 111 that is smaller than the distal portion inside diameter 113. Located between the proximal portion proximal end 106 and the distal portion distal end 102, the implantable device 100 may have a proximal portion 322, a tapered transition portion 308, and a distal portion 338. In one application, for example, in a dialysis application, the proximal portion proximal end 106 may be in communication with an artery and the distal portion distal end 102 may be in communication with a vein. Alternatively, the proximal portion proximal end 106 may be in communication with a vein and the distal portion distal end 102 may be in communication with an artery. In other examples, the implantable device 100 may consist entirely of a transition portion 308, meaning the implantable device 100 is tapered entirely along its length between the proximal end 106 and the distal end 102.

The transition portion 308 has a transition portion length 324 between a transition portion proximal end 304 and transition portion distal end 300. The transition portion 308 may comprise a first (inner) layer 310 having a transition portion first layer wall thickness 312. The transition portion first layer wall thickness 312 may vary along the transition portion length 324. The transition portion first layer wall thickness 312 may be thicker on the transition portion proximal end 304 than the transition portion first layer wall thickness 312 at the transition portion distal end 300. Alternatively, the transition portion first layer wall thickness 312 may be thinner on the transition portion proximal end 304 than the transition portion first layer wall thickness 312 at the transition portion distal end 300.

The first layer 310 has a first layer first surface 358 and a first layer second surface 360, as shown in Figures 3A-C. The first layer first surface 358 and the first layer second surface 360 may extend along the implantable device 100 (e.g., along the proximal portion 322, transition portion 308, and the distal portion 338 or a combination thereof). As shown by example in Figures 3A-C, the first layer first surface 358 and the first layer second surface 360 extend along the proximal portion 322, transition portion 308, and the distal portion 338.

The transition portion 308 may also have an intermediate layer 314 adjacent to at least a portion of the first layer 310. For example, as shown in Figures 3A-C, the transition portion 308 has an intermediate layer 314 adjacent to or juxtaposed to the first layer second surface 360. Alternatively, the intermediate layer 314 may be partially imbibed into porous first layer 310 near the interface of those two layers. In one example, the intermediate layer 314 is an elastomer (e.g., silicone), and the first layer 310 is a porous polymer (e.g., ePTFE).

The intermediate layer 314 has a transition portion intermediate layer wall thickness 316 that varies along the transition portion 308. The transition portion intermediate layer wall thickness 316 may taper between the transition portion proximal end 304 and the transition portion distal end 300 as shown in Figures 3A-B. The transition portion intermediate layer wall thickness 316 may be thinner near the transition portion proximal end 304 than the transition portion distal end 300. Alternatively, the transition portion intermediate layer wall thickness 316 may be thicker at the transition portion proximal end 304 than the transition portion distal end 300.

The intermediate layer 314 may also be juxtaposed to an optional additional layer (e.g., second layer 318). Alternatively, the intermediate layer 314 may be partially imbibed into another layer, for example, the second porous layer 318, in the surface region of the second layer 318 that is adjacent to the intermediate layer 314. The intermediate layer 314 may extend proximally and/or distally beyond the transition portion 308.

The transition portion 308 may have a second (outer) layer 318. For example, as shown in Figures 3A-C, the transition portion 308 has a second layer 318, adjacent to the intermediate layer 314, with a transition portion second layer wall thickness 320. The transition portion second layer wall thickness 320 may taper between the transition portion proximal end 304 and the transition portion distal end 300, as shown in Figures 3A-C. The transition portion intermediate layer wall thickness 316 may be thicker near the transition portion proximal end 304 than near the transition portion distal end 300. Alternatively, the transition portion proximal end 304 may be thinner than the transition portion distal end 300. The second layer 318 may extend proximally and/or distally beyond the transition portion 308.

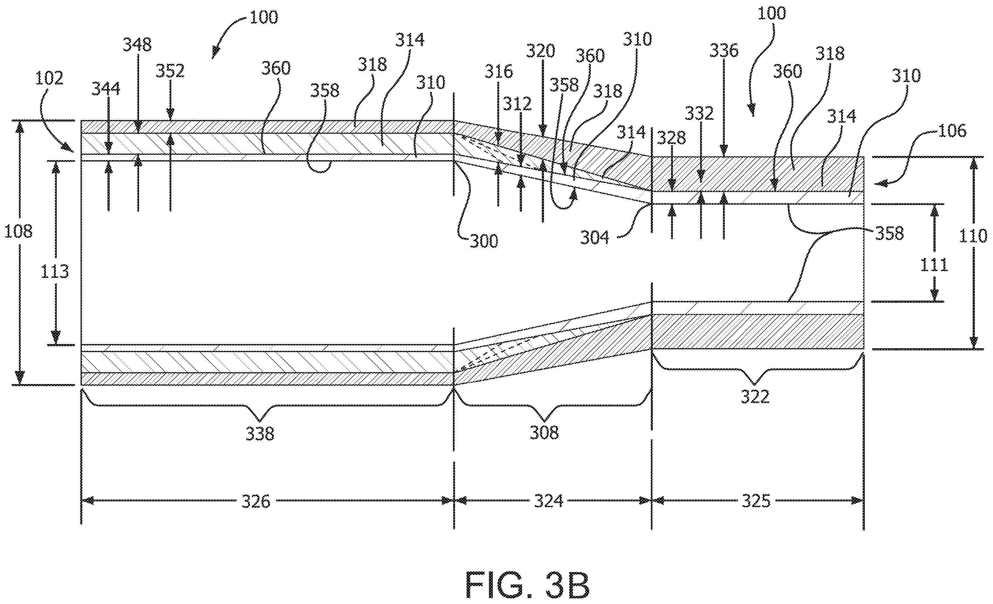

The intermediate layer 314 tapers between the transition portion proximal end 304 and the transition portion distal end 300. The **i**ntermediate layer 314 tapers such that the intermediate layer wall thickness 316 is zero or approximately zero at any point within the transition portion length 324 (as is represented by the dotted lines showing the boundary of the intermediate layer 314). In certain instances, the intermediate layer 314 tapers such that the intermediate layer wall thickness 316 is zero or approximately zero the at the transition portion proximal end 304. As shown in Figure 3B, the intermediate layer 314 tapers at a constant rate toward the transition portion proximal end 304. In certain instances, the taper of the intermediate layer wall thickness 316 between the transition portion proximal end 304 and the transition portion distal end 300 may be non-constant and may increase or decrease in rate as it approaches the transition portion proximal end 304. As shown in Figure 3B, the proximal portion 322 does not include the intermediate layer 314. The lack of the intermediate layer 314 being present in the proximal portion 322 (and/or the decrease in thickness of the intermediate layer 314 in the transition portion length 324) may enhance the ease of puncture of the proximal portion 322 (or the transition portion length 324) by a suture (or the like) to secure the implantable device 100 to a patient. In certain instances, the transition portion second layer wall thickness 320 may increase in thickness toward the transition portion proximal end 304 as the intermediate layer wall thickness 316 decreases in thickness (e.g., as shown in Figure 3B). In other instances, the transition portion first layer wall thickness 312 may increase in thickness toward the transition portion proximal end 304 as the intermediate layer wall thickness 316 decreases in thickness. In other instances, an overall wall thickness of the implantable device 100 within the transition portion length 324 may decrease as the intermediate layer wall thickness 316 decreases.

Also as shown in Figures 3A-C, the implantable device 100 may have a distal portion 338 between the transition portion distal end 300 and a distal portion distal end 102. The distal portion 338 may have varying diameters (inside and/or outside) along the distal portion 338 or it may have constant diameters. For example, the distal portion 338 has a constant distal portion outer diameter 108 between the distal portion distal end 102 and the transition portion distal end 300, as shown in Figures 3A-C. The distal portion 338 may have a constant distal portion inside diameter 113 between the distal portion distal end 102 and the transition portion distal end 300. The distal portion 338 may comprise a first layer 310 having a distal portion first layer wall thickness 344, an intermediate layer 314 having a distal portion intermediate layer wall thickness 348, and a second layer 318 having a distal portion second layer wall thickness 352.

Also as shown in Figures 3A-C, the implantable device 100 may have a proximal portion 322 between a proximal portion proximal end 106 and transition portion proximal end 304. The proximal portion 322 may have varying diameters (inside and/or outside) along the proximal portion 322 or it may have constant diameters. For example, the proximal portion 322 has the same proximal portion outer diameter 110 between the proximal portion proximal end 106 and the transition portion proximal end 304, as shown in Figures 3A-C. The proximal portion 322 may have a proximal portion inside diameter 111 that is constant between the proximal portion proximal end 106 and the transition portion proximal end 304 or alternatively, may have a varying proximal portion inside diameter 111. The proximal portion 322 may comprise a proximal portion first layer 310 having a proximal portion first layer wall thickness 328, a proximal portion intermediate layer 314 having a proximal portion intermediate layer wall thickness 332, and a proximal portion second layer 318 having a proximal portion second layer wall thickness 336.

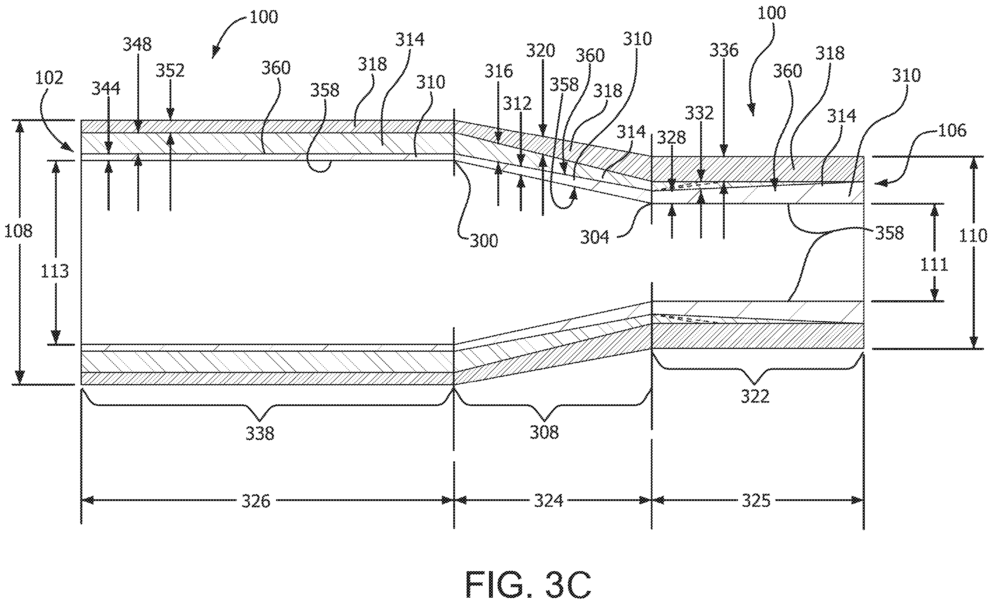

The proximal portion intermediate layer 314 tapers between the transition portion proximal end 304 and the proximal end 106. The intermediate layer 314 tapers such that the intermediate layer wall thickness 332 is zero or approximately zero at any point within the proximal portion length 325 (as is represented by the dotted lines showing the boundary of the intermediate layer 314). In certain instances, the intermediate layer 314 tapers such that the intermediate layer wall thickness 332 is zero or approximately zero the at the proximal end 106. The decrease in thickness of the intermediate layer 314 in the proximal portion 322 enhances the ease of puncture of the proximal portion 322 by a suture (or the like) to secure the implantable device 100 to a patient. As shown in Figure 3C, the intermediate layer 314 tapers at a constant rate toward the proximal end 304. In certain instances, the taper of the intermediate layer wall thickness 332 between the transition portion proximal end 304 and the proximal end 106 may be non-constant and may increase or decrease as it approaches the proximal end 106. In certain instances, the proximal portion first layer wall thickness 328 may increase in thickness toward the proximal end 106 as the proximal portion intermediate layer 314 decreases in thickness (e.g., as shown in Figure 3C). In other instances, the proximal portion second layer wall thickness 336 may increase in thickness toward the proximal end 106 as the intermediate layer wall thickness 332 decreases in thickness. In other instances, an overall wall thickness of the implantable device 100 within the proximal portion length 325 may decrease as the intermediate layer wall thickness 332 decreases.

The transition portion 308 has a transition portion proximal end 304 (Figure 5) with a transition portion proximal end outer diameter 306 and a transition portion proximal end inside diameter 307. The implantable device 100 also has a transition portion distal end 300 (Figure 4) with a transition portion distal end outer diameter 302, and a transition portion distal end inside diameter 303. The transition portion distal end diameters (302,303) may be the same diameter as distal portion distal end diameters (108,113). The transition portion proximal end diameters (306,307) may be the same diameter as proximal portion proximal end diameters (110,111).

The proximal portion 322 may have various wall thickness configurations. In one example, as shown in Figure 3A, the proximal portion 322 has a first layer 310, an intermediate layer 314, and a second layer 318. The first layer 310 has a proximal portion first layer wall thickness 328 that is substantially the same thickness along the proximal portion 322 and meets the transition portion first layer wall thickness 312 at the transition portion proximal end 304. The intermediate layer 314 has a proximal portion intermediate layer wall thickness 332 that is substantially the same thickness along the proximal portion 322 and meets the transition portion intermediate layer wall thickness 316 at the transition portion proximal end 304. The second layer 318 has a proximal portion second layer wall thickness 336 that is substantially the same thickness along the proximal portion 322 and meets the transition portion second layer wall thickness 320 at the transition portion proximal end 304. Alternatively, the proximal portion 322 may have wall thicknesses that are not constant along the proximal portion length 325.

The distal portion 338 may have various wall thickness configurations. In one example, as shown in Figures 3A-C, the distal portion 338 has a first layer 310, an intermediate layer 314, and a second layer 318. The first layer 310 has a distal portion first layer wall thickness 344 that is substantially the same thickness along the distal portion 338 and meets the transition portion first layer wall thickness 312 at the transition portion distal end 300. The intermediate layer 314 has a distal portion intermediate layer wall thickness 348 that is substantially the same thickness along the distal portion 338 and meets the transition portion intermediate layer wall thickness 316 at the transition portion distal end 300. The second layer 318 has a distal portion second layer wall thickness 352 that is substantially the same thickness along the distal portion 338 and meets the transition portion second layer wall thickness 320 at the transition portion distal end 300. Alternatively, the distal portion 338 may have wall thicknesses that are not constant along the distal portion length 326.

The implantable device 100 may have various overall wall thickness configurations. For example, as shown in Figure 4, the transition portion 308 (also shown in Figures 3A-C) may have a transition portion distal end overall wall thickness 354 at the transition portion distal end 300 that is less than a transition portion proximal end overall wall thickness 356 at the transition portion proximal end 304. The transition portion overall wall thickness at a location along the transition portion may vary between transition portion overall wall thicknesses (354,356).

An overall wall thickness measured along the implantable device 100 may comprise at least one polymer layer (e.g., ePTFE) and at least one elastomer layer (e.g., silicone). The implantable device 100 may have a transition portion 308 comprising a transition portion overall wall thickness 354 (as shown in Figure 4). A transition portion wall thickness ratio, taken along the transition portion 308 (e.g., at transition portion distal end 300), of transition portion intermediate layer wall thickness 316 to a combination of transition portion first layer wall thickness 312 and transition portion second layer wall thickness 320 is equal to 1. In other words, this ratio is the ratio of the intermediate layer wall thickness to the combination of the inner and outer layer wall thicknesses. In other cases, the transition portion distal end wall thickness ratio may be greater than 1.0, for example, 1.1, 1.2, 1.3, 1.4, 1.5 or even greater. Still, in other cases, the transition portion wall thickness ratio may be less than 1.0, for example, 0.7, 0.8, or 0.9.

The implantable device 100 may also have a transition portion 308 comprising a transition portion overall wall thickness 356 (Figure 5). A transition portion wall thickness ratio, taken along the transition portion 308 (e.g., at transition portion proximal end 304), of the transition portion intermediate layer wall thickness 316 to a combination of the transition portion first layer wall thickness 312 and the transition portion second layer wall thickness 320 is less than 1. For example, the transition portion proximal end ratio of the transition portion intermediate layer wall thickness 316 to a combination of the transition portion first layer wall thickness 312 and the transition portion second layer wall thickness 320 may be 0.9, 0.8, 0.7, 0.6, 0.5, 0.4 or less. The transition portion proximal end ratio may extend along the implantable device 100 to a proximal portion 322, as shown in Figure 3A. Similarly, the transition portion distal end ratio may extend along the implantable device 100 to a distal portion 338, also as shown in Figure 3A.

In addition to the ratios described above, the overall wall thickness 356 at the transition portion proximal end 304 may be greater than the overall wall thickness 354 at the transition portion distal end 300.

The implantable device 100 may have various portions. For example, it may have a proximal portion 322 with a proximal portion length 325, a transition portion 308 with a transition portion length 324, and a distal portion 338 with a distal portion length 326, as shown in Figures 3A-C. The proximal portion length 325 may be shorter than the transition portion length 324 and the transition portion length 324 may be shorter than the distal portion length 326. The distal portion length 326 may be 15cm, 20cm, 30cm, 40cm, 50cm or more, and the transition portion length 324 may be 5cm ( other lengths may range from 1cm to 10cm), and the proximal portion length 325 may be approximately 2cm (other lengths may range from 1cm to 10cm). Other combinations of lengths are contemplated and dependent upon application. In some cases, the implantable device 100 may be entirely a transition portion 308 (i.e., the entire graft is tapered) and therefore may have a transition portion length 324 of 20cm, 30cm, 40cm, or 50cm or more.

An implantable device 100 may be manufactured in various ways. One example is described herein, as follows. A 145cm long mandrel having a 7mm diameter portion by 120cm long, a 3.84mm diameter portion by 20cm long, and a 5cm long tapered portion between the 7mm diameter portion and the 3.84mm diameter portion was obtained (i.e., a 7-4mm tapered mandrel). A 3.60mm diameter extruded ePTFE tube with a wall thickness of 0.08mm was obtained (ePTFE first tube). The ePTFE first tube has a lumen extending along the ePTFE first tube. The 7-4mm tapered mandrel was then inserted into the ePTFE first tube. The ePTFE first tube was extended so that any wrinkling of the ePTFE first tube was minimized. The ePTFE first tube was then helically wrapped with a 1.27cm wide ePTFE film tape entirely along the length of the ePTFE first tube. The mandrel with the ePTFE first tube was then heated in a convection oven at 370 degrees C for 12 minutes. Following removal from the oven, the ePTFE first tube on the 7-4mm tapered mandrel was then allowed to air cool in ambient air until the ePTFE tube reached approximately room temperature. This example produced an ePTFE first tube with approximately a 7mm inside diameter length portion, a 4mm inside diameter length portion, and a tapered length portion extending between the 7mm inside diameter length portion and the 4mm inside diameter length portion. The 7mm inside diameter length portion had a wall thickness of approximately 0.08mm and the 4mm inside diameter length portion had a wall thickness of approximately 0.10mm. The tapered length portion had a varying wall thickness (i.e., varying from 0.08mm to 0.10mm) between the 7mm inside diameter length portion and the 4mm inside diameter length portion.

A second ePTFE tube with a taper was manufactured as follows. A 145cm long mandrel with a 6.50mm diameter portion by 110cm long, a 3.80mm diameter portion by 30cm long, and a 5cm long tapered length portion between the 6.50mm diameter length portion and the 3.80mm diameter length portion was obtained (6.5 -3.80mm tapered mandrel). A 6mm diameter extruded and expanded ePTFE tube with a wall thickness of 0.66mm was obtained (ePTFE second tube). The 6.50-3.80mm tapered mandrel was inserted into the ePTFE second tube lumen. The ePTFE second tube was extended so that any wrinkling of the ePTFE second tube was minimized. The ends of the ePTFE second tube were then secured to the mandrel by wrapping an ePTFE film around the ends of the ePTFE second tube. The ePTFE second tube was then helically wrapped with a 1.90cm wide ePTFE film tape entirely along the length of the ePTFE second tube. The mandrel with the ePTFE second tube was then heated by a convection oven at 370 degrees C for 12 minutes. Following removal from the oven, the ePTFE second tube on the mandrel was then allowed to air cool in ambient air until the ePTFE second tube and mandrel reached approximately room temperature. The ePTFE second tube was then removed from the mandrel. This procedure resulted in an ePTFE second tube with a 6.5mm inside diameter length portion, a 3.80mm inside diameter length portion, and a tapered length portion extending between the 6.50mm inside diameter length portion and the 3.80mm inside diameter length portion. The 6.50mm inside diameter length portion had a wall thickness of approximately 0.76mm and the 3.80mm inside diameter length portion had a wall thickness of approximately 0.79mm. The tapered length portion had a varying wall thickness that tapered between the wall thickness of the 6.50mm inside diameter length portion and the wall thickness of the 3.80mm inside diameter length portion. In certain instances, the tapered length portion had a varying wall thickness that tapered between the wall thickness of the 6mm inside diameter length portion and the wall thickness of the 4mm inside diameter length portion, and in other instances, the tapered length portion had a varying wall thickness that tapered between the wall thickness of the 5mm inside diameter length portion and the wall thickness of the 4mm inside diameter length portion.

The mandrel with the ePTFE first tube was then coated with a layer of silicone (NuSil Technology LLC, Carpinteria, CA). An iris apparatus (Standa Ltd., Vilnius, Lithuania) capable of dilating and contracting between 1mm and 15mm was obtained. The mandrel with the first ePTFE tube was displaced through the iris contained within an apparatus. The apparatus supplied silicone to the outer surface of the ePTFE first tube and the iris maintained a wall thickness of silicone of approximately 0.53mm on the ePTFE first tube outer surface (along the larger diameter portion of the ePTFE first tube), 0.30mm on the smaller diameter portion of the ePTFE first tube, and a varying silicone wall thickness between 0.53mm and 0.30mm along the tapered portion of the ePTFE first tube. The silicone was then allowed to partially set by curing in a convection oven for 6.5 minutes at 200 degrees C.

The ePTFE second tube was then enlarged diametrically by pulling the ePTFE second tube over a mandrel. The mandrel had a 9.60mm outside diameter length portion of approximately 55cm length and a 5.40mm outside diameter length portion of 12cm length with a transition length portion extending between the 5.40mm outside diameter length portion and the 9.60mm outside diameter length portion. The ePTFE second tube and mandrel assembly was then heated in a convection oven for approximately 45 seconds at 200 degrees C. The ePTFE second tube distal end inside diameter was enlarged to approximately 9.60mm over the ePTFE second tube distal end and the ePTFE second tube proximal end inside diameter was enlarged to approximately 5.40mm on the ePTFE second tube proximal end with the transition length portion extending between the 9.60mm inside diameter and the 5.40mm inside diameter.

After the ePTFE second tube was removed from the mandrel, a flare of 12mm was then created on the ePTFE second tube distal end (i.e., 9.60mm inside diameter end) with a flaring tool (a mandrel). The ePTFE second tube was then held within the apparatus with the iris to allow the ePTFE first tube with its outer layer of silicone to be inserted into the lumen of the ePTFE second tube. The ePTFE first tube proximal portion was inserted first through the ePTFE second tube distal portion until the transition length portions of the ePTFE first tube and ePTFE second tube were approximately aligned. While the ePTFE first tube was being inserted through the ePTFE second tube the apparatus applied a second layer of silicone approximately 0.15mm thick (the second layer being thinner than the first layer) onto the ePTFE first tube with silicone outer layer. The second layer of silicone wall thickness was controlled by the iris apparatus. After the second layer of silicone was applied and the ePTFE first tube and ePTFE second tube tapered portions were aligned, the silicone was then allowed to fully set by curing in a convection oven for 15 minutes at 200 degrees C. In this example, an implantable device according to this disclosure was made.

The resulting implantable device had a first layer comprising an ePTFE first (Inner) tube, an intermediate layer comprising silicone, and a second layer comprising an ePTFE second (outer) tube. The implantable device had a transition length portion with a length of 5cm, a distal length portion with a length of 48cm and a proximal length portion with a length of 2cm. Longitudinal cross sections were taken and wall thickness measurements were made (overall wall thickness and intermediate layer wall thickness) with an optical profiler (Unitron Inc., Bohemia NY) along the proximal length portion and the distal length portion of the implantable device. The overall wall thickness in the proximal length portion was approximately 1.25mm. The intermediate layer wall thickness in the proximal length portion was approximately 0.48mm. The overall wall thickness in the distal length portion was approximately 1.22mm and the intermediate layer wall thickness in the distal length portion was 0.70mm. The overall wall thickness in the transition length portion varied from 1.25mm near the proximal length portion to 1.22mm near the distal length portion and the intermediate layer wall thickness in the transition length portion varied from 0.48mm near the proximal length portion to 0.70mm near the distal length portion. A ratio of intermediate layer wall thickness to a combination of the ePTFE first tube wall thickness and ePTFE second tube wall thickness in the proximal length portion was approximately 0.63. A ratio of intermediate layer wall thickness to a combination of the ePTFE first tube wall thickness and ePTFE second tube wall thickness in the distal length portion was approximately 1.35mm.

Implantable devices according to this disclosure may be used in various applications. For example, the implantable device may be used in an arterial venous application such as in dialysis. One end of the implantable device may be attached to a vein, an opposing end may be attached to an artery.

In addition to the teachings described above and claimed below, devices and/or methods having different combinations of the features described above and claimed below are contemplated. As such, the description is also directed to other devices and/or methods having any other possible combination of the dependent features claimed below.

Numerous characteristics and advantages have been set forth in the preceding description, including various alternatives together with details of the structure and function of the devices and/or methods. The disclosure is intended as illustrative only and as such is not intended to be exhaustive. It will be evident to those skilled in the art that various modifications may be made, especially in matters of structure, materials, elements, components, shape, size and arrangement of parts including combinations within the principles of the invention, to the full extent indicated by the broad, general meaning of the terms in which the appended claims are expressed. To the extent that these various modifications do not depart from the scope of the appended claims, they are intended to be encompassed therein.

## Claims

1. An implantable device (100) with a distal end (102) and a proximal end (106) comprising:
a tubular structure having a transition portion (308) having a transition portion proximal end (304) with a transition portion proximal end inside diameter (111), a transition portion distal end (300) with a transition portion distal end inside diameter (113) that is greater than the transition portion proximal end inside diameter (111), and a transition portion length (324) between the transition portion proximal end (304) and the transition portion distal end (300);
wherein the tubular structure has a first layer (310) comprising a porous polymer and at least one intermediate layer (314) adjacent the first layer (310);
wherein the intermediate layer (314) has a variable wall thickness extending at least partially along the transition portion length (324), wherein the intermediate layer (314) has an intermediate layer wall thickness (316) that tapers from the transition portion distal end (300) to the transition portion proximal end (304), and the intermediate layer wall thickness (316) of the intermediate layer (314) goes to approximately zero before the proximal end (106), and
wherein the intermediate layer (314) comprises an elastomer.

2. The implantable device (100) of claim 1 further comprising a second layer (318).

3. The implantable device (100) of claim 1 wherein the first layer (310) is ePTFE.

4. The implantable device (100) of claim 1 wherein the intermediate layer (314) has an intermediate layer wall thickness (348) at the transition portion distal end (300) that is greater than an intermediate layer wall thickness (332) at the transition portion proximal end (304).

5. The implantable device (100) of claim 1 wherein the intermediate layer (314) is silicone.

6. The implantable device (100) of claim 1 wherein the distal portion (338) has a length (326) between the transition portion distal end (300) and a distal portion distal end (102) that is greater than the transition portion length (324) and the transition portion length (324) is greater than a proximal portion length (325) between a proximal portion proximal end (106) and the transition portion proximal end (304).

7. The implantable device (100) of claim 2 wherein the second layer (318) is ePTFE.

8. The implantable device (100) of claim 1 wherein the intermediate layer wall thickness (316) of the intermediate layer (314) at the proximal end (106) is zero.

9. The implantable device (100) of claim 1, wherein the intermediate layer (314) has a transition portion intermediate layer wall thickness (316) that varies along the transition portion (308) wherein the transition portion intermediate layer wall thickness (316) tapers such that the transition portion intermediate layer wall thickness (316) is thinner near the transition portion proximal end (304) than the transition portion distal end (300).

10. The implantable device (100) of claim 1, wherein the transition portion first layer (310) has a transition portion first layer wall thickness (312) that varies along the transition portion length (324) such that the first layer wall thickness (312) is thicker on the transition portion proximal end (304) than the transition portion distal end (300).

11. The implantable device (100) of claim 1, wherein the second layer (318) has a transition portion second layer wall thickness (320) that varies between the transition portion proximal end (304) and the transition portion distal end (300) such that a proximal portion second layer wall thickness (336) increases in thickness toward the proximal end (106).

## Patentansprüche

1. Implantierbare Vorrichtung (100) mit einem distalen Ende (102) und einem proximalen Ende (106), umfassend:
eine rohrförmige Struktur, die einen Übergangsabschnitt (308) aufweist, der ein proximales Ende (304) des Übergangsabschnittes mit einem Innendurchmesser (111) des proximalen Endes des Übergangsabschnittes, ein distales Ende (300) des Übergangsabschnittes mit einem Innendurchmesser (113) des distalen Endes des Übergangsabschnittes, der größer als der Innendurchmesser (111) des proximalen Endes des Übergangsabschnittes ist, und eine Übergangsabschnittslänge (324) zwischen dem proximalen Ende (304) des Übergangsabschnittes und dem distalen Ende (300) des Übergangsabschnittes aufweist;
wobei die rohrförmige Struktur eine erste Schicht (310), die ein poröses Polymer umfasst, und zumindest eine Zwischenschicht (314) benachbart zu der ersten Schicht (310) aufweist;
wobei die Zwischenschicht (314) eine variable Wanddicke aufweist, die sich zumindest teilweise entlang der Übergangsabschnittslänge (324) erstreckt, wobei die Zwischenschicht (314) eine Zwischenschichtwanddicke (316) aufweist, die sich von dem distalen Ende (300) des Übergangsabschnittes zu dem proximalen Ende (304) des Übergangsabschnittes verjüngt, und die Zwischenschichtwanddicke (316) der Zwischenschicht (314) vor dem proximalen Ende (106) auf ungefähr Null geht, und
wobei die Zwischenschicht (314) ein Elastomer umfasst.

2. Implantierbare Vorrichtung (100) nach Anspruch 1, ferner umfassend eine zweite Schicht (318).

3. Implantierbare Vorrichtung (100) nach Anspruch 1, wobei die erste Schicht (310) ePTFE ist.

4. Implantierbare Vorrichtung (100) nach Anspruch 1, wobei die Zwischenschicht (314) eine Zwischenschichtwanddicke (348) an dem distalen Ende (300) des Übergangsabschnittes aufweist, die größer als eine Zwischenschichtwanddicke (332) an dem proximalen Ende (304) des Übergangsabschnittes ist.

5. Implantierbare Vorrichtung (100) nach Anspruch 1, wobei die Zwischenschicht (314) Silikon ist.

6. Implantierbare Vorrichtung (100) nach Anspruch 1, wobei der distale Abschnitt (338) eine Länge (326) zwischen dem distalen Ende (300) des Übergangsabschnittes und einem distalen Ende (102) des distalen Abschnittes aufweist, die größer als die Übergangsabschnittslänge (324) ist, und die Übergangsabschnittslänge (324) größer als eine Länge (325) des proximalen Abschnittes zwischen einem proximalen Ende (106) des proximalen Abschnittes und dem proximalen Ende (304) des Übergangsabschnittes ist.

7. Implantierbare Vorrichtung (100) nach Anspruch 2, wobei die zweite Schicht (318) ePTFE ist.

8. Implantierbare Vorrichtung (100) nach Anspruch 1, wobei die Zwischenschichtwanddicke (316) der Zwischenschicht (314) an dem proximalen Ende (106) Null ist.

9. Implantierbare Vorrichtung (100) nach Anspruch 1, wobei die Zwischenschicht (314) eine Übergangsabschnittszwischenschichtwanddicke (316) aufweist, die entlang des Übergangsabschnittes (308) variiert, wobei sich die Übergangsabschnittszwischenschichtwanddicke (316) verjüngt, sodass die Übergangsabschnittszwischenschichtwanddicke (316) nahe dem proximalen Ende (304) des Übergangsabschnittes dünner als dem distalen Ende (300) des Übergangsabschnittes ist.

10. Implantierbare Vorrichtung (100) nach Anspruch 1, wobei die erste Schicht (310) des Übergangsabschnittes eine Wanddicke (312) der ersten Schicht des Übergangsabschnittes aufweist, die entlang der Übergangsabschnittslänge (324) variiert, sodass die Wanddicke (312) der ersten Schicht an dem proximalen Ende (304) des Übergangsabschnittes dicker als dem distalen Ende (300) des Übergangsabschnittes ist.

11. Implantierbare Vorrichtung (100) nach Anspruch 1, wobei die zweite Schicht (318) eine Wanddicke (320) der zweiten Schicht des Übergangsabschnittes aufweist, die zwischen dem proximalen Ende (304) des Übergangsabschnittes und dem distalen Ende (300) des Übergangsabschnittes variiert, sodass eine Wanddicke (336) der zweiten Schicht des proximalen Abschnittes in der Dicke zu dem proximalen Ende (106) zunimmt.

## Revendications

1. Dispositif implantable (100) doté d'une extrémité distale (102) et d'une extrémité proximale (106) comprenant :
une structure tubulaire comportant une partie de transition (308) possédant une extrémité proximale de partie de transition (304) avec un diamètre interne d'extrémité proximale de partie de transition (111), une extrémité distale de partie de transition (300) avec un diamètre interne d'extrémité distale de partie de transition (113) qui est supérieur au diamètre interne d'extrémité proximale de partie de transition (111), et une longueur de partie de transition (324) entre l'extrémité proximale de partie de transition (304) et l'extrémité distale de partie de transition (300) ;
ladite structure tubulaire comportant une première couche (310) comprenant un polymère poreux et au moins une couche intermédiaire (314) adjacente à la première couche (310) ;
ladite couche intermédiaire (314) présentant une épaisseur de paroi variable s'étendant au moins partiellement le long de la longueur de partie de transition (324), ladite couche intermédiaire (314) présentant une épaisseur de paroi de couche intermédiaire (316) qui se rétrécit de l'extrémité distale de partie de transition (300) à l'extrémité proximale de partie de transition (304), et ladite épaisseur de paroi de couche intermédiaire (316) de la couche intermédiaire (314) tendant approximativement vers zéro avant l'extrémité proximale (106), et
ladite couche intermédiaire (314) comprenant un élastomère.

2. Dispositif implantable (100) selon la revendication 1, comprenant en outre une seconde couche (318).

3. Dispositif implantable (100) selon la revendication 1, ladite première couche (310) étant en PTFEe.

4. Dispositif implantable (100) selon la revendication 1, ladite couche intermédiaire (314) présentant une épaisseur de paroi de couche intermédiaire (348) au niveau de l'extrémité distale de partie de transition (300) qui est supérieure à l'épaisseur de paroi de couche intermédiaire (332) au niveau de l'extrémité proximale de partie de transition (304).

5. Dispositif implantable (100) selon la revendication 1, ladite couche intermédiaire (314) étant en silicone.

6. Dispositif implantable (100) selon la revendication 1, ladite partie distale (338) présentant une longueur (326) entre l'extrémité distale de partie de transition (300) et une extrémité distale de partie distale (102) qui est supérieure à la longueur de partie de transition (324) et ladite longueur de partie de transition (324) étant supérieure à une longueur de partie proximale (325) entre une extrémité proximale de partie proximale (106) et l'extrémité proximale de partie de transition (304).

7. Dispositif implantable (100) selon la revendication 2, ladite seconde couche (318) étant en PTFEe.

8. Dispositif implantable (100) selon la revendication 1, ladite épaisseur de paroi de couche intermédiaire (316) de la couche intermédiaire (314) au niveau de l'extrémité proximale (106) étant nulle.

9. Dispositif implantable (100) selon la revendication 1, ladite couche intermédiaire (314) présentant une épaisseur de paroi de couche intermédiaire de partie de transition (316) qui varie le long de la partie de transition (308), ladite épaisseur de paroi de couche intermédiaire de partie de transition (316) se rétrécissant de sorte que l'épaisseur de paroi de couche intermédiaire de partie de transition (316) soit plus mince près de l'extrémité proximale de partie de transition (304) que l'extrémité distale de partie de transition (300).

10. Dispositif implantable (100) selon la revendication 1, ladite première couche (310) de partie de transition présentant une épaisseur de paroi de première couche de partie de transition (312) qui varie le long de la longueur de partie de transition (324) de sorte que l'épaisseur de paroi de première couche (312) soit plus épaisse sur l'extrémité proximale de partie de transition (304) que l'extrémité distale de partie de transition (300).

11. Dispositif implantable (100) selon la revendication 1, ladite seconde couche (318) présentant une épaisseur de paroi de seconde couche de partie de transition (320) qui varie entre l'extrémité proximale de partie de transition (304) et l'extrémité distale de partie de transition (300) de sorte qu'une épaisseur de paroi de seconde couche de partie proximale (336) augmente en épaisseur en direction de l'extrémité proximale (106).
